# EUROPEAN PATENT APPLICATION

(11) **EP 4 338 780 A1**
(43) Date of publication of application: **20.03.2024**
(21) Application number: 22807463.9
(22) Date of filing: 10.05.2022
(51) Int. Cl.: A61M 25/00, A61M 25/14

(54) **CATHETER**

(30) Priority: 12.05.2021 JP 2021081278
(71) Applicant: Asahi Intecc Co., Ltd., Seto-shi, Aichi 489-0071 (JP)
(72) Inventor: KOJIMA Ryoutarou, Seto-shi, Aichi 489-0071 (JP); KANEKO Yoshiki, Seto-shi, Aichi 489-0071 (JP)
(74) Representative: Winter, Brandl - Partnerschaft mbB
(86) International application number: PCT/JP2022/019783
(87) International publication number: WO 2022/239763

(57) **Abstract**

A catheter 10 of the disclosed embodiments include a shaft 1 having lumens 2, 3 inside the shaft, and a reinforcing body 6 that is inserted in the shaft 1 and extended along the lumens 2, 3. The reinforcing body 6 includes a first portion (main body part) 61, and a second portion (distal end portion) 62 that is positioned on the more distal end side than the first portion 61 while being at a distal end portion of the shaft 1 and has larger direct or indirect joining force relative to the shaft 1 than the first portion 61. Such a catheter is able to increase the breaking strength.

## Description

### [Technical Field]

The disclosed embodiments relate to a catheter.

### [Background Art]

The catheter used in treating a site in a body cavity such as a blood vessel is required to be thinner and more flexible toward the distal end in order to enter the inside of the thin and bending body cavity. To be thin and flexible, the tensile strength tends to be normally reduced. Thus, there is known the technology of improving the breaking strength of a shaft part and suppressing the extension thereof by disposing a core wire in the catheter. For example, Patent Literature 1 discloses the catheter including the distal end side shaft formed of a resin tube, a rear end side shaft having higher rigidity than the distal end side shaft, and a guide wire lumen, in which a core wire for adjusting flexibility is disposed in the shaft and fixed to the distal end side shaft at a position near the rear end side opening portion of the guide wire lumen, thereby increasing the breaking strength of the resin tube and controlling the extension thereof.

### [Citation List]

### [Patent Literature]

[Patent Literature 1] JP 2008-125897 A

### [Summary of Invention]

### [Technical Problem]

However, in the catheter in Patent Literature 1, the strength of a part on the more distal end side than the part where the core wire is provided is not secured, and a break may occur at such a part. Moreover, even in a case where the core wire is extended to the distal end side of the catheter to avoid a break at such a part, there is a problem that the extending and breaking strength of the members other than the core wire is not secured because the core wire and the other members are not integrated.

In view of such an aspect, the disclosed embodiments aim at providing a catheter with the improved breaking strength.

### [Solution to Problem]

In order to achieve the above-described object, the present embodiments provide a catheter, including: a shaft that includes a lumen inside the shaft, and a reinforcing body that is inserted in the shaft and extended along the lumen, in which the reinforcing body includes a first portion, and a second portion that is positioned on the more distal end side than the first portion while being at a distal end portion of the shaft and has larger direct or indirect joining force relative to the shaft than the first portion (Embodiment 1).

In such an embodiment (Embodiment 1), even the distal end portion of the shaft is reinforced by the reinforcing body, which improves the breaking strength of the entire shaft. In addition, the second portion on the shaft distal end portion side of the reinforcing body is strongly joined to the shaft, and thus it is possible to prevent the reinforcing body from being removed from the shaft when tension is imposed in the longitudinal direction of the catheter, which improves the breaking strength of the catheter.

The above-described embodiment (Embodiment 1) further includes a cylindrical member that is provided on the outer peripheral surface of the lumen at the distal end portion of the shaft, in which the second portion may be fixed to the cylindrical member (Embodiment 2).

In such an embodiment (Embodiment 2), even the distal end portion of the shaft is reinforced by the reinforcing body, which improves the breaking strength of the entire shaft. In addition, the second portion on the shaft distal end portion side of the reinforcing body is joined to the cylindrical member in the shaft, and thus it is possible to prevent the reinforcing body from being removed from the shaft when tension is imposed in the longitudinal direction of the catheter, which improves the breaking strength of the catheter.

In the above-described embodiments (Embodiments 1, 2), the second portion may include an engagement portion that has a larger cross sectional area than the first portion (Embodiment 3).

In the above-described embodiments (Embodiments 1 to 3), the lumen is a first lumen, a second lumen is further provided, and the first portion may be formed to have, at least on the distal end portion side of the shaft, a flat shape in a direction where the first lumen and the second lumen are provided in parallel to each other (Embodiment 4).

In such an embodiment (Embodiment 4), the catheter including therein two lumens is able to suppress increase of bending rigidity at the distal end portion.

### [Advantageous Effects of Invention]

The catheter of the disclosed embodiments secure the improvement of breaking strength by the presence of a reinforcing body.

### [Brief Description of Drawings]

[Fig. 1] Fig. 1 is an explanatory view illustrating a structure of a catheter according to one of the disclosed embodiments.
[Fig. 2] Fig. 2 is an explanatory view illustrating a structure of a distal end portion of the catheter.
[Fig. 3] Fig. 3 is a sectional view cut along A-A' line of Fig. 2.
[Fig. 4] Fig. 4 is an explanatory view illustrating a modification of a structure of a distal end portion of the catheter.

### [Description of Embodiments]

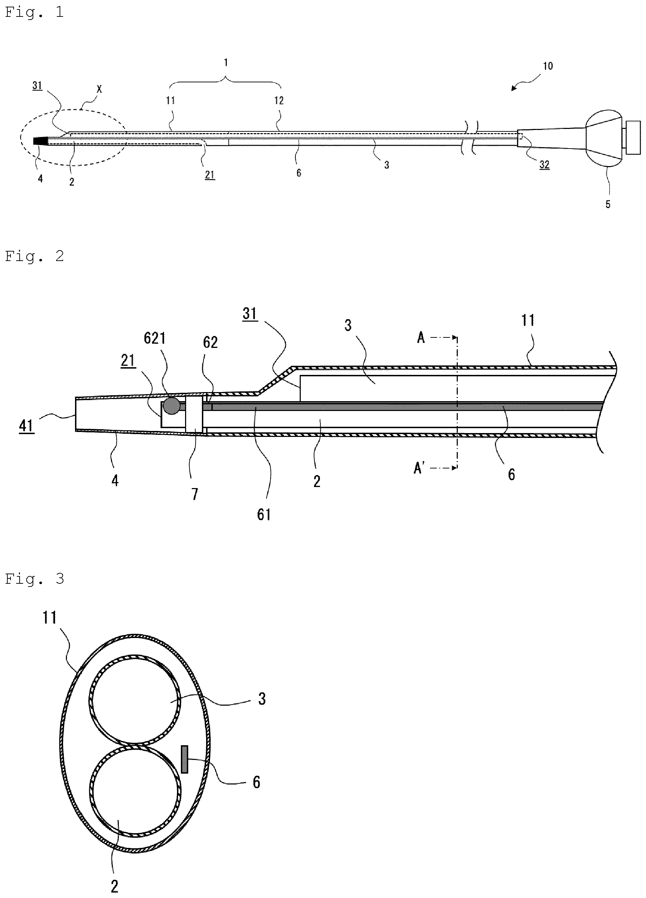

Hereinafter, a disclosed embodiments will be described with reference to the drawings. Fig. 1 is an explanatory view illustrating a structure of a catheter 10 according to the embodiment. Moreover, Fig. 2 is an explanatory view illustrating a structure of a distal end portion (X portion in Fig. 1) of the catheter 10, and Fig. 3 is a sectional view cut along A-A' line of Fig. 2. Note that the disclosed embodiments are not limited to ones described in the following, which are merely examples to illustrate the technical features of the disclosed embodiments. Moreover, the shape and size in each drawing are merely illustrated to facilitate understanding of the contents of the disclosed embodiments, and do not precisely reflect the actual shape and size.

Note that in the specification, the "distal end side" is a direction along the axial direction of a shaft, and refers to a direction along which the catheter is advanced toward a treatment site. The "proximal end side" is a direction along the axial direction of a shaft, and refers to a direction opposite to the above-described distal end side. Moreover, the "distal end" refers to an end on the distal end side of an arbitrary member or portion, and the "proximal end" refers to an end on the proximal end side of an arbitrary member or portion. Furthermore, the "distal end portion" refers to a portion, in an arbitrary member or portion, including the distal end and extending from the distal end toward the proximal end side to the halfway of the above-described member or the like, and the "proximal end portion" refers to a portion, in an arbitrary member or portion, including the proximal end and extending from the proximal end toward the distal end side to the halfway of the above-described member or the like.

As illustrated in Fig. 1, the catheter 10 includes a shaft 1 having therein a first lumen 2 and a second lumen 3, and a distal tip 4 is attached to the distal end of the shaft 1, and a connector 5 is attached to the proximal end of the shaft 1. Moreover, the catheter 10 includes therein a core wire (reinforcing body) 6 that is inserted in the shaft 1 and extended along the first lumen 2 and the second lumen 3. The shaft 1 is a long tubular member having, along the axial direction, the first lumen 2 and the second lumen 3 in which a guide wire is inserted, and the shape (length and diameter) of the shaft 1 is appropriately designed according to the intended purpose and use position.

The shaft 1 includes a distal end side shaft 11 and a proximal end side shaft 12. The first lumen 2 and the second lumen 3 are provided in parallel to each other in the distal end side shaft 11, while the only second lumen 3 is extended from the distal end side shaft 11 and provided in the proximal end side shaft 12. The distal end side shaft 11 and the proximal end side shaft 12 may be configurated by different materials or the same material.

Note that in the embodiment, the catheter 10 is described as a double-lumen catheter for penetration that includes two lumens (first lumen 2 and second lumen 3) in each of which a guide wire is inserted, the first lumen 2 being an RX (rapid exchange) type guide wire lumen and the second lumen 3 being an OTW (over the wire) type guide wire lumen. However, the catheter of the disclosed embodiments are not limited to this, and the implementation of the disclosed embodiments is possible as long as the catheter includes therein at least one lumen. The catheter of the disclosed embodiments may be, for example, a single lumen catheter with only one lumen, a guiding catheter, a balloon catheter, or the like.

As illustrated in Figs. 1 and 2, in the first lumen 2, an opening 21 on the distal end side is disposed in the distal tip 4 attached to the distal end of the distal end side shaft 11 and connected to the distal tip 4, and an opening 22 on the proximal end side is disposed on a surrounding wall in the vicinity of the proximal end of the distal end side shaft 11 and is open to the outside of the catheter 10. Moreover, in the second lumen 3, an opening 31 on the distal end side is disposed in the vicinity of the distal end portion of the distal end side shaft 11, and an opening 32 on the proximal end side is connected to the connector 5 attached to the proximal end of the proximal end side shaft 12.

Materials configurating the shaft 1, the first lumen 2, and the second lumen 3 are not particularly limited, but are preferably antithrombotic, flexible, and biocompatible because they are to be inserted into a body cavity. As such materials, it is possible to adopt, for example, resin materials or the like such as polyamide, polyamide elastomer, polyolefin, polyester, polyester elastomer, polyurethane, silicone, and fluorocarbon resin.

The distal tip 4 is a cylindrical (hollow-shaped) member connected to the distal end of the shaft 1 (distal end side shaft 11). The distal tip 4 includes, for example, an inner cavity 41 penetrating along the axial direction, and can be formed in a tapered shape in which that the diameter is substantially reduced toward the distal end side.

The material configurating the distal tip 4 may be a more flexible material than the material configurating the shaft 1, and the examples include resin materials or the like such as polyurethane, polyurethane elastomer, polyamide, and polyamide elastomer. Moreover, to enable visual recognition upon X-ray irradiation, these resin materials may be mixed with powder such as tungsten.

The method of joining the distal end side shaft 11 and the proximal end side shaft 12, and the method of joining the distal tip 4 and the distal end side shaft 11 are not particularly limited unless the effects of the disclosed embodiments are deteriorated. It is possible to adopt, for example, a method of welding resin materials by heating, a method of adhering them by an adhesive, or the like.

The connector 5 is a member for the professional to hold the catheter 1, and is connected to the proximal end of the shaft 1. The connector 5 includes an inner cavity (not illustrated) formed to penetrate along the axial direction, and the second lumen 3 is communicated to the distal end side opening of the inner cavity.

The core wire 6 is a reinforcing body that is inserted in the shaft 1 and extended along the first lumen 2 and the second lumen 3, and is a rod-like member having a longitudinal shape as a whole. The core wire 6 extends over the entire length of the shaft 1 from the proximal end portion to the distal end portion, and as illustrated in Fig. 3, the sectional shape is formed to be a rectangular flat shape, that is, a flat linear shape. Note that the core wire 6 has a rectangular flat shape in order to intentionally form a direction easy to bend. In the example of Fig. 3, the core wire 6 is inserted to be parallel to a virtual line (not illustrated) connecting the centers of the first lumen 2 and the second lumen 3.

The core wire 6 includes a main body part 61 as a first portion, and a distal end portion 62 as a second portion that is positioned on the more distal end side than the main body part 61 while being at the distal end portion of the shaft 1, and is configurated so that the direct or indirect joining force of the distal end portion 62 relative to the shaft 1 is larger than that of the main body part 61 relative to the shaft 1.

Materials configurating the core wire 6 preferably has excellent rigidity from the viewpoint of preventing a cut of the core wire 6 itself and rotating the distal end portion of the catheter 10 securely and precisely. Examples of the material include metallic materials or the like such as stainless steel including SUS304, a nickel titanium alloy, and a cobalt chrome alloy.

In the distal tip 4 connected to the distal end of the shaft 1, there is provided an impermeable marker 7 formed of a material through which X-rays are hardly transmittable, on the outer peripheral surface of the distal end portion of the first lumen 2. The impermeable marker 7 is a metallic member having a cylindrical shape, and is disposed to surround the outer peripheral surface of the distal end portion of the first lumen 2. The impermeable marker 7 is formed of, for example, metal such as gold, platinum, and tungsten. Thus, when inserting the catheter 10 into the living body, it is possible to image the position of the impermeable marker 7 with X-rays from the outside of the living body.

In the embodiment, a spherical engagement portion 621 engaged with the shaft 1 is formed at the distal end of the distal end portion 62 of the core wire 6. The spherical engagement portion 621 is formed to have a larger sectional shape than the flat linear part other than the engagement portion 621 in the core wire 6. That is, the distal end portion 62 includes the engagement portion 621 having a larger cross sectional area than the main body part 61. Moreover, the distal end portion 62 of the core wire is disposed on the inner side of the impermeable marker 7, that is, disposed to be sandwiched between the impermeable marker 7 and the outer peripheral surface of the first lumen 2, and the impermeable marker 7 is caulked, thereby fixing the distal end portion 62 of the core wire 6 to the impermeable marker 7. In this manner, the distal end portion of the core wire 6 is fixed to the shaft 1.

In the catheter 10 described above, even the distal end portion of the shaft 1 is reinforced by the core wire 6 as a reinforcing body, which improves the breaking strength of the entire shaft 1. In addition, the end portion (distal end portion 62) on the shaft distal end portion side of the core wire 6 is strongly joined to the shaft 1, and thus it is possible to prevent the core wire 6 from being removed from the shaft when tension is imposed in the longitudinal direction of the catheter 10, which improves the breaking strength of the catheter 10. Especially in the embodiment, the engagement portion 621 is provided at the distal end of the distal end portion 62 of the core wire 6 such that it is positioned on the more distal end side than the impermeable marker 7, and the core wire 6 is sandwiched between the impermeable marker 7 and the outer peripheral surface of the first lumen 2. This configuration allows the engagement portion 621 to be caught by the impermeable marker 7 and prevents the core wire 6 from being easily removed from the shaft 1.

Moreover, the catheter that includes the shaft 1 having therein two lumens (first lumen 2 and second lumen 3) provided in parallel to each other, such as the catheter 10, has characteristics of easily bending in the right and left direction in Fig. 3 and hardly bending in the up and down direction. Here, in a case where the core wire 6 is formed to be flat in the direction where the two lumens (first lumen 2 and second lumen 3) are provided in parallel to each other (up and down direction in Fig. 1 to Fig. 3) at least on the distal end portion side of the shaft 1, it is possible to suppress increase of bending rigidity at the distal end portion of the shaft 1.

### <Modification>

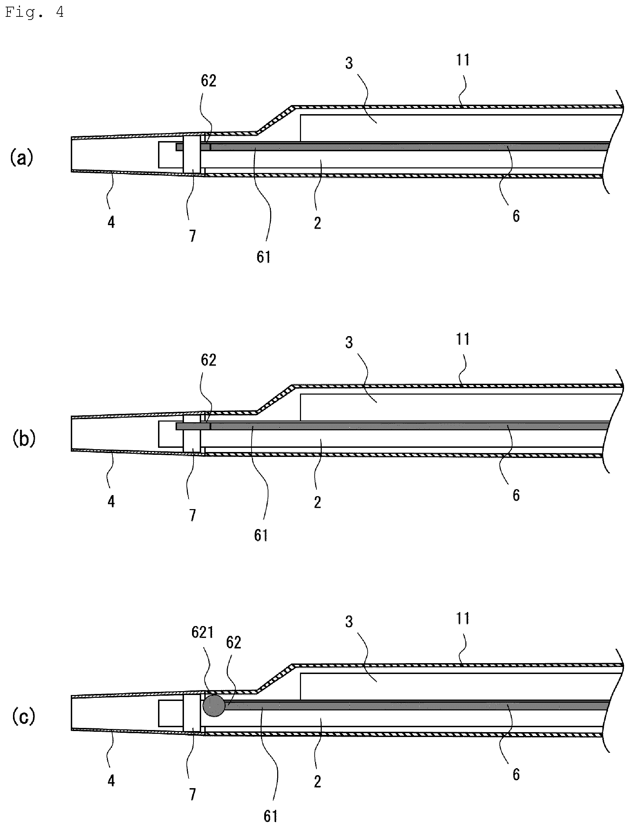

Hereinafter, modifications of the structure of the distal end portion of the catheter 10 according to the embodiment are described with reference to Fig. 4. In any of the modifications, the core wire 6 includes the main body part 61 as a first portion, and the distal end portion 62 as a second portion that is positioned on the more distal end side than the main body part 61 while being at the distal end portion of the shaft 1, and is configurated so that the direct or indirect joining force of the distal end portion 62 relative to the shaft 1 is larger than that of the main body part 61 relative to the shaft 1.

In a modification illustrated in Fig. 4(a), an engagement portion is not formed at the distal end of the distal end portion 62 of the core wire 6. However, the distal end portion 62 of the core wire is disposed on the inner side of the impermeable marker 7, and the impermeable marker 7 is caulked, thereby fixing the distal end portion 62 of the core wire 6 to the impermeable marker 7. In this manner, with the distal end portion 62 of the core wire 6 fixed to the shaft 1, it is possible to prevent the core wire 6 from being removed from the shaft when tension is imposed in the longitudinal direction of the catheter 10.

In a modification illustrated in Fig. 4(b), the distal end portion 62 of the core wire is disposed on the outer side of the impermeable marker 7, and the distal end portion 62 is welded to the outer peripheral surface of the impermeable marker 7, thereby fixing the distal end portion 62 of the core wire 6 to the impermeable marker 7. In this manner, with the distal end portion 62 of the core wire 6 fixed to the shaft 1, it is possible to prevent the core wire 6 from being removed from the shaft when tension is imposed in the longitudinal direction of the catheter 10.

In a modification illustrated in Fig. 4(c), the spherical engagement portion 621 engaged with the shaft 1 is formed at the distal end portion 62 of the core wire 6, and the engagement portion 621 is positioned in the vicinity of the impermeable marker 7. Even in a case where the distal end portion 62 of the core wire 6 is not fixed to the impermeable marker 7, it is possible to fix the distal end portion 62 of the core wire 6 to the shaft 1 by the engagement portion 621 formed to be caught between the inner peripheral surface of the distal end side shaft 11 and the outer peripheral surface of the first lumen 2. In this manner, with the distal end portion 62 of the core wire 6 fixed to the shaft 1, it is possible to prevent the core wire 6 from being removed from the shaft when tension is imposed in the longitudinal direction of the catheter 10.

The above has described the catheter according to the disclosed embodiments with reference to the drawings. However, the disclosed embodiments are not limited to the above-described ones, and various changes can be made. For example, the engagement portion 621 formed at the distal end portion 62 of the core wire 6 is not limited to have a spherical shape, but may have a block shape with a larger sectional shape than the flat linear part other than the engagement portion 621 in the core wire 6, a hook shape, or any shape allowing fixing to the inside of the shaft and thus exerting the anchor effect.

Moreover, the method of fixing the distal end portion 62 of the core wire 6 to the inside of the shaft 1 may be a method of welding the distal end portion 62 of the core wire 6 to the inner peripheral surface of the impermeable marker 7, a method of fixing the distal end portion 62 of the core wire 6 provided with the engagement portion 621 to the inner peripheral surface or the outer peripheral surface of the impermeable marker 7 by welding, a method of fixing the distal end portion 62 of the core wire 6 disposed on the inner side of the impermeable marker 7 to the inner peripheral surface of the impermeable marker 7 by welding, and further caulking the impermeable marker 7, or a method of fixing the distal end portion 62 of the core wire 6 to the impermeable marker 7 by an adhesive or the like.

Furthermore, in a case where a reinforcing layer is formed using a metallic coil member or the like on the outer peripheral surface of the first lumen 2 or the second lumen 3 to prevent crushing, the distal end portion 62 of the core wire 6 may be fixed to the reinforcing layer provided on the lumen so as to fix the distal end portion 62 of the core wire 6 to the inside of the shaft 1.

### [Reference Signs List]

- 10: catheter
- 1: shaft
- 11: distal end side shaft
- 12: proximal end side shaft
- 2: first lumen
- 3: second lumen
- 4: distal tip
- 5: connector
- 6: core wire (reinforcing body)
- 61: main body part (first portion)
- 62: distal end portion (second portion)
- 621: engagement portion
- 7: impermeable marker (cylindrical member)

## Claims

1. A catheter, comprising:
a shaft that includes a lumen inside the shaft; and
a reinforcing body that is inserted in the shaft and extended along the lumen, wherein
the reinforcing body includes a first portion, and a second portion that is positioned on the more distal end side than the first portion while being at a distal end portion of the shaft and has larger direct or indirect joining force relative to the shaft than the first portion.

2. The catheter according to claim 1, further comprising:
a cylindrical member that is provided on an outer peripheral surface of the lumen at the distal end portion of the shaft, wherein
the second portion is fixed to the cylindrical member.

3. The catheter according to claims 1 or 2, wherein
the second portion includes an engagement portion that has a larger cross sectional area than the first portion.

4. The catheter according to any one of claims 1 to 3, wherein
the lumen is a first lumen,
a second lumen is further provided, and
the first portion is formed to have, at least on the distal end portion side of the shaft, a flat shape in a direction where the first lumen and the second lumen are provided in parallel to each other.
